(19) Europäisches Patentamt
European Patent Office
Office européen des brevets

(11) EP 1 129 065 B1

(12) # FASCICULE DE BREVET EUROPEEN

(45) Date de publication et mention de la délivrance du brevet:
**21.01.2004 Bulletin 2004/04**

(21) Numéro de dépôt: **99971795.2**

(22) Date de dépôt: **09.11.1999**

(51) Int Cl.$^7$: **C07C 213/02**

(86) Numéro de dépôt international:
**PCT/FR1999/002745**

(87) Numéro de publication internationale:
**WO 2000/027796 (18.05.2000 Gazette 2000/20)**

(54) **PROCEDE DE PREPARATION DE TRIS(ETHER-AMINE)**

VERFAHREN ZUR HERSTELLUNG VON TRIS(ÄTHER-AMINEN)

METHOD FOR PREPARING TRIS(ETHER-AMINE)

(84) Etats contractants désignés:
**AT BE CH CY DE DK ES FI FR GB GR IE IT LI LU MC NL PT SE**

(30) Priorité: **09.11.1998 FR 9814081**

(43) Date de publication de la demande:
**05.09.2001 Bulletin 2001/36**

(73) Titulaire: **RHODIA CHIMIE**
**92512 Boulogne Billancourt Cedex (FR)**

(72) Inventeurs:
- **ALAS, Michel**
  **F-79500 Melle (FR)**
- **BOUNIOT, Albert**
  **F-79500 Paizay le Tort (FR)**

(74) Mandataire: **Bernasconi, Jean Raymond et al**
**c/o Cabinet Lavoix,**
**2, Place d'Estienne d'Orves**
**75441 Paris Cedex 09 (FR)**

(56) Documents cités:
**EP-A- 0 005 094**
**EP-A- 0 018 884**
**WO-A-88/06579**
**US-A- 2 285 419**

- **G RARD SOULA: "Tris(polyoxaalkyl)amines (Trident), anew class of solid-liquid phase-transfer catalysts" JOURNAL OF ORGANIC CHEMISTRY., vol. 50, no. 20, 1985, pages 3717-3721, XP002105964 EASTON US**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 129 065 B1

Printed by Jouve, 75001 PARIS (FR)

## Description

La présente invention concerne un procédé amélioré de préparation d'amines tertiaires utilisables comme agents sequestrants pour solubiliser les sels métalliques organiques ou minéraux dans les solvants organiques. Ces amines tertiaires peuvent également être utilisées comme émulsifiants.

Les amines tertiaires préparées selon le procédé de l'invention ont pour formule générale (I) :

$$(I) \qquad N[A\text{-}O\text{-}(B\text{-}O)_n\text{-}R]_3$$

dans laquelle :

R représente un radical alkyle présentant de 1 à 24 atomes de carbone, éventuellement substitué par un ou plusieurs radicaux alcoxy en $C_1$-$C_{12}$ ; un radical carbocyclique, monocyclique ou polycyclique, saturé, présentant de 3 à 10 atomes de carbone, éventuellement substitué par un ou plusieurs radicaux alcoxy en $C_1$-$C_{12}$ ; un radical alkyle présentant de 1 à 12 atomes de carbone et porteur d'un groupe carbocyclique, monocyclique ou polycyclique, saturé, présentant de 3 à 10 atomes de carbone, la partie alkyle étant éventuellement substituée par un ou plusieurs radicaux alcoxy en $C_1$-$C_{12}$ et le groupe carbocyclique saturé étant éventuellement substitué par un ou plusieurs groupes alkyle en $C_1$-$C_{12}$ ou alcoxy en $C_1$-$C_{12}$ ; un radical carbocyclique aromatique, monocyclique ou polycyclique, présentant de 6 à 22 atomes de carbone et éventuellement substitué par un ou plusieurs groupes $C_1$-$C_{12}$ alcoxy ou $C_1$-$C_{12}$ alkyle ; et un radical alkyle présentant de 1 à 12 atomes de carbone et porteur d'un groupe carbocyclique aromatique, monocyclique ou polycyclique, ayant de 6 à 18 atomes de carbone, la partie alkyle étant éventuellement substituée par un ou plusieurs groupes alcoxy en $C_1$-$C_{12}$ et le groupe aromatique étant éventuellement substitué par un ou plusieurs alkyle en $C_1$-$C_{12}$ ou alcoxy en $C_1$-$C_{12}$ ;
A et B, identiques ou différents, représentent indépendamment une chaîne alkylène linéaire présentant de 1 à 24 atomes de carbone éventuellement substituée par un ou plusieurs groupes choisis parmi alkyle en $C_1$-$C_{12}$ et alcoxy en $C_1$-$C_{12}$ ; et
n représente de 0 à 12.

L'intérêt de telles amines fait notamment l'objet de la demande de brevet FR 79 05438.

De nombreux procédés de préparation ont été décrits dans la technique permettant la préparation de telles amines tertiaires. On se reportera par exemple à EP161459, EP5094, EP18884 ainsi qu'aux travaux de Pétrov parus dans Zh. Obsch. Khim (1970), 40(7), 1611-1616.

Cependant les procédés décrits présentent deux inconvénients majeurs : les rendements sont faibles et la purification des produits difficile.

Du point de vue des rendements obtenus, la méthode de synthèse décrite dans EP18884 est particulièrement avantageuse. Elle consiste à mettre en oeuvre l'ammonolyse, en phase liquidé, d'un monoéther d'alkylèneglycol de formule F1 :

$$HO - A - O - (B\text{-}O)_n\text{-}R \qquad F1$$

dans laquelle :

R représente $(C_1$-$C_{24})$alkyle, cyclohexyle, phényle ou $(C_1$-$C_{12})$ alkylphényle;
A et B identiques ou différents, représentent indépendamment une chaîne $(C_2$-$C_3)$ alkylène linéaire dans laquelle les atomes de carbone sont éventuellement substitués par méthyle ou éthyle; et
n représente un nombre entier compris entre 0 et 4;

en présence d'un catalyseur d'hydrogénation-déshydrogénation entre 100 et 250°C, l'opération d'ammonolyse étant réalisée par mise en contact du ou des agents d'ammonolyse avec un mélange constitué du monoéther d'alkylèneglycol ci-dessus et du catalyseur.

Les agents d'ammonolyse sont, selon ce document, choisis parmi l'ammoniac et des éther-amines.de formule F2 :

$$H_{3-p}N\ [A\text{-}O(B\text{-}O)_n\text{-}R]_p \qquad F2$$

dans laquelle :

R représente ($C_1$-$C_{24}$)alkyle, cyclohexyle, phényle ou ($C_1$-$C_{12}$) alkylphényle;
A et B identiques ou différents, représentent indépendamment une chaîne ($C_2$-$C_3$) alkylène linéaire dans laquelle les atomes de carbone sont éventuellement substitués par méthyle ou éthyle; et
n représente un nombre entier compris entre 0 et 4 ; et
p est un entier égal à 1 ou 2.

Malgré le faible coût des matières premières, ce procédé n'est pas économique. En fin de réaction, le milieu réactionnel est séparé par filtration du catalyseur. Or, l'opération de filtration n'est pas aisée et nécessite des installations de filtration adaptées qui sont coûteuses aussi bien à l'achat qu'à l'entretien, le catalyseur d'hydrogénation - déshydrogénation étant pyrophorique et abrasif.
D'autre part, l'élimination du catalyseur est laborieuse et souvent incomplète. On observe ainsi une diminution notable du rendement dans la mesure où des traces de catalyseurs dans l'amine tertiaire brute provoquent sa décomposition lors des étapes ultérieures de purification par distillation.
Enfin, la technique de l'art antérieur est peu appropriée à l'industrialisation du procédé puisqu'elle nécessiterait la mise en oeuvre de quantités excessives de catalyseur, lequel est particulièrement coûteux.
Le procédé de l'invention vise à pallier les inconvénients du procédé de l'art antérieur.
Plus précisément, l'invention concerne un procédé de préparation de tris(éther-amines) de formule (1) telle que définies ci-dessus comprenant la réaction, en phase liquide, d'un monoéther d'alkylèneglycol de formule (II) :

(II) $HO\text{-}A\text{-}O\text{-}(B\text{-}O)_n\text{-}R$

dans laquelle :

R représente un radical alkyle présentant de 1 à 24 atomes de carbone, éventuellement substitué par un ou plusieurs radicaux alcoxy en $C_1$-$C_{12}$ ; un radical carbocyclique, monocyclique ou polycyclique, saturé, présentant de 3 à 10 atomes de carbone, éventuellement substitué par un ou plusieurs radicaux alcoxy en $C_1$-$C_{12}$ ; un radical alkyle présentant de 1 à 12 atomes de carbone et porteur d'un groupe carbocyclique, monocyclique ou polycyclique, saturé, présentant de 3 à 10 atomes de carbone, la partie alkyle étant éventuellement substituée par un ou plusieurs radicaux alcoxy en $C_1$-$C_{12}$ et le groupe carbocyclique saturé étant éventuellement substitué par un ou plusieurs groupes alkyle en $C_1$-$C_{12}$ ou alcoxy en $C_1$-$C_{12}$ ; un radical carbocyclique aromatique, monocyclique ou polycyclique, présentant de 6 à 22 atomes de carbone et éventuellement substitué par un ou plusieurs groupes $C_1$-$C_{12}$ alcoxy ou $C_1$-$C_{12}$ alkyle ; et un radical alkyle présentant de 1 à 12 atomes de carbone et porteur d'un groupe carbocyclique aromatique, monocyclique ou polycyclique, ayant de 6 à 18 atomes de carbone, la partie alkyle étant éventuellement substituée par un ou plusieurs groupes alcoxy en $C_1$-$C_{12}$ et le groupe aromatique étant éventuellement substitué par un ou plusieurs alkyle en $C_1$-$C_{12}$ ou alcoxy en $C_1$-$C_{12}$ ;
A et B, identiques ou différents, représentent indépendamment une chaîne alkylène linéaire présentant de 1 à 24 atomes de carbone éventuellement substituée par un ou plusieurs groupes choisis parmi alkyle en $C_1$-$C_{12}$ et alcoxy en $C_1$-$C_{12}$ ; et
n représente de 0 à 12 ;

avec un agent d'ammonolyse choisi parmi l'ammoniac et une éther-amine de formule (I') :

(I') $H_{3-p}N\,[A\text{-}O\text{-}(B\text{-}O)_n\text{-}R]_p$

où A, B, n et R sont tels que définis ci-dessus pour la formule (I) et p présente 1 ou 2, à une température comprise entre 100 et 250°C par mise en contact des réactifs avec un catalyseur d'hydrogénation-déshydrogénation.
Selon l'invention, on entend par radical carbocyclique, monocyclique ou polycyclique saturé, un radical constitué d'un ou plusieurs noyaux cycloalkyle. Lorsque ledit radical carbocyclique saturé comprend plusieurs noyaux cycloalkyle, ceux-ci forment des structures condensées ou pontées, ce qui signifie que chaque noyau cycloalkyle a en commun avec au moins un autre noyau cycloalkyle, au moins deux atomes de carbone.
Des exemples de structures condensées sont notamment le perhydronaphtalène et le perhydroindane.
De même, un exemple de structure pontée est le norbornane.
On préfère cependant les radicaux carbocycliques monocycliques saturés de type ($C_3$-$C_8$)cycloalkyle tels que cyclopropyle, cyclobutyle, cyclopentyle, cyclohexyle, cycloheptyle ou cyclooctyle.

Dans le cadre de l'invention, les radicaux alkyle sont linéaires ou ramifiés. A titre de radicaux alkyle préférés, on peut citer les radicaux $C_1$-$C_{10}$ alkyle, mieux encore ($C_1$-$C_6$)alkyle, et notamment méthyle, éthyle, propyle, isopropyle, butyle, isobutyle, sec-butyle, tert-butyle, pentyle et hexyle.

Les radicaux carbocycliques aromatiques sont monocycliques ou polycycliques.

Les radicaux carbocycliques aromatiques polycycliques présentant des noyaux aromatiques condensés.

Parmi ceux-ci on préfère les radicaux carbocycliques aromatiques mono- et bicycliques présentant de 6 à 10 atomes de carbone tels que phényle et naphtyle.

Des exemples particuliers de radicaux alkyle porteurs d'un radical carbocyclique aromatique sont les groupes ($C_6$-$C_{10}$)aryl-($C_1$-$C_{12}$)alkyle et plus particulièrement les groupes ($C_6$-$C_{10}$)aryl-($C_1$-$C_6$)alkyle tels que benzyle ou naphtylméthyle.

Dans le cadre de l'invention, les définitions préférées données ci-dessus pour les groupes alkyle, les radicaux carbocycliques saturés ou aromatiques restent des significations préférées de ces groupes lorsque ceux-ci font partie intégrante de radicaux alcoxy ou bien alkyle porteur d'un radical carbocyclique saturé ou aromatique.

Le procédé de l'invention est plus particulièrement approprié à la préparation des sous-groupes suivants des composés de formule I.

Un premier sous-groupe est constitué des composés de formule I dans laquelle :

R représente ($C_1$-$C_6$)alkyle éventuellement substitué par ($C_1$-$C_6$)alcoxy ; ($C_3$-$C_8$)cycloalkyle éventuellement substitué par un ou plusieurs groupes ($C_1$-$C_6$)alcoxy ; ($C_6$-$C_{10}$)aryle éventuellement substitué par un ou plusieurs groupes ($C_1$-$C_6$)alcoxy ; ($C_6$-$C_{10}$)aryl-($C_1$-$C_6$)alkyle dans lequel la partie alkyle est éventuellement substituée par ($C_1$-$C_6$)alcoxy et la partie aryle est éventuellement substituée par ($C_1$-$C_6$)alkyle ou ($C_1$-$C_6$)alcoxy ; ou ($C_3$-$C_8$) cycloalkyl-($C_1$-$C_6$)alkyle dans lequel la partie alkyle est éventuellement substituée par un ou plusieurs ($C_1$-$C_6$) alcoxy et la partie cycloalkyle est éventuellement substituée par ($C_1$-$C_6$)alkyle ou ($C_1$-$C_6$)alcoxy;
A et B, identiques ou différents, représentent une chaîne ($C_1$-$C_6$)alkylène linéaire éventuellement substituée par un ou plusieurs groupes ($C_1$-$C_6$)alkyle ou ($C_1$-$C_6$)alcoxy ; et
n représente de 0 à 6.

Un second groupe de composés préférés est constitué des composés de formule I dans laquelle :

R représente ($C_1$-$C_{24}$)alkyle, cyclohexyle, phényle ou ($C_1$-$C_{12}$) alkylphényle;
A et B identiques ou différents, représentent indépendamment une chaîne ($C_2$-$C_3$) alkylène linéaire dans laquelle les atomes de carbone sont éventuellement substitués par méthyle ou éthyle; et
n représente un nombre entier compris entre 0 et 4.

Un troisième groupe de composés préférés est constitué des composés de formule I dans laquelle :

R représente ($C_1$-$C_6$)alkyle, cyclohexyle, phényle ou ($C_1$-$C_6$)alkylphényle ;
A et B identiques ou différents, représentent indépendamment une chaîne ($C_2$-$C_3$)alkylène linéaire dans laquelle les atomes de carbone sont éventuellement substitués par méthyle ou éthyle, et
n représente un nombre entier compris entre 0 et 4.

Le procédé de l'invention est caractérisé en ce que la mise en contact est réalisée en faisant passer une solution desdits réactifs, préalablement portée à une température de 100 à 250°C à travers un lit catalytique constitué de particules dudit catalyseur d'hydrogénation-déshydrogénation.

Les réactifs mis en jeu dans le procédé de l'invention sont le monoéther d'alkylèneglycol de formule (II) :

$$HO\text{-}A\text{-}O\text{-}(B\text{-}O)_n\text{-}R \qquad (II)$$

où A, B, n et R sont tels que définis ci-dessus pour la formule (I) et l'agent d'ammonolyse qui est choisi parmi l'ammoniac et une éther-amine de formule (I') :

$$H_{3-p}N[A\text{-}O\text{-}(B\text{-}O\ )_n\text{-}R]_p$$

où A, B, n, R et p sont tels que définis ci-dessus. Selon le procédé de l'invention, on prépare une solution des réactifs que l'on porte à une température comprise entre 100 et 250°C, de préférence entre 100 et 180°C.

Lorsque l'agent d'ammonolyse est l'ammoniac, celui-ci est mis en solution dans le monoéther d'alkylèneglycol de formule (II) : ceci peut être simplement réalisé par dissolution d'ammoniac gazeux dans une solution du monoéther d'alkylèneglycol.

Lorsque l'agent d'ammonolyse est l'amine primaire de formule

$$H_2N[A\text{-}O(B\text{-}O)_n\text{-}R]$$

ou l'amine secondaire de formule:

$$HN[A\text{-}O\text{-}(B\text{-}O)_n\text{-}R]_2$$

où A, B, n et R sont tels que définis ci-dessus, la solution des réactifs est simplement préparée par mélange des réactifs.

Les amines primaires préférées utilisables dans le procédé de l'invention sont choisies parmi :

- l'oxa-3 butylamine
- l'oxa-3 pentylamine
- l'oxa-3 hexylamine
- l'oxa-3 heptylamine
- la dioxa-3,6 heptylamine
- la trioxa-3,6,9 undécylamine
- la dioxa-3,6 octylamine
- la trioxa-3,6,9 dodécylamine
- la dioxa-3,6 nonylamine
- la trioxa-3,6,9 tridécylamine
- la dioxa-3,6 décylamine
- la trioxa-3,6,9 tétradécylamine
- la phénoxy-5 oxa-3 pentylamine
- la phénoxy-8 dioxa-3,6 octylamine
- la cyclohexoxy-5 oxa-3 pentylamine
- la cyclohexoxy-8 dioxa-3,6 octylamine
- la nonylphénoxy-5 oxa-3 pentylamine
- la nonylphénoxy-8 dioxa-3,6 octylamine
- la dodécylphénoxy-5 oxa-3 pentylamine
- la dodécylphénoxy-8 dioxa-3,6 octylamine
- la dioxa-3,6 méthyl-4 heptylamine
- la dioxa-3,6 diméthyl-2,4 heptylamine.

Les amines secondaires préférées utilisables dans le procédé de l'invention sont choisies parmi :

- l'aza-5 dioxa-2,8 nonane
- l'aza-8 tétraoxa-2,5,11,14 pentadécane
- l'aza-11 hexaoxa-2,5,8,14,17,20 uneicosane
- l'aza-6 dioxa-3,9 undécane
- l'aza-10 tétraoxa-4,7,13,16 nonadécane
- l'aza-9 tétraoxa-3,6,12,15 heptadécane
- l'aza-12 hexaoxa-3,6,9, 15,18,21 tricosane
- l'aza-7 dioxa-4,10 tridécane
- l'aza-13 hexaoxa-4,7,10,16,19,22 pentacosane
- l'aza-8 dioxa-5,11 pentadécane
- l'aza-11 tétraoxa-5,8,14,17 uneicosane
- l'aza-14 hexaoxa-5,8,11,17,20,23 heptacosane
- l'aza-6 oxa-3 phénoxy-1 undécane
- l'aza-9 dioxa-3,6 phénoxy-1 heptadécane
- l'aza-6 oxa-3 cyclohexoxy-1 undécane
- l'aza-9 dioxa-3,6 cyclohexoxy-1 heptadécane

- l'aza-6 oxa-3 nonylphénoxy-1 undécane
- l'aza-9 dioxa-3,6 nonylphénoxy-1 heptadécane
- l'aza-6 oxa-3 dodécylphénoxy-1 undécane
- l'aza-9 dioxa-3,6 dodécylphénoxy heptadécane
- l'aza-8 tétraoxa-2,5,11,14 diméthyl-4,12 pentadécane, et
- l'aza-8 tétraoxa-2,5,11,14 tétraméthyl-4,6,10,12 pentadécane.

La solution des réactifs peut comprendre en plus des réactifs ci-dessus un ou plusieurs solvants inertes vis-à-vis de la réaction d'ammonolyse.

De tels solvants sont par exemple des hydrocarbures aliphatiques, des hydrocarbures aromatiques ou leurs mélanges, dont la température d'ébullition sous pression atmosphérique est comprise entre 100 et 350° C.

La solution des réactifs est ensuite mise en contact avec le catalyseur par passage de cette solution à travers un lit catalytique constitué dudit catalyseur d'hydrogénation-déshydrogénation.

Selon un mode de réalisation de l'invention, la solution des réactifs est préparée de façon extemporanée en amont du lit catalytique.

Toutefois, lorsque l'agent d'ammonolyse est l'ammoniac, il est également possible de préparer la solution des réactifs en entrée du lit catalytique par injection d'un flux gazeux d'ammoniac, dans un courant entrant de la solution de monoéther d'alkylèneglycol.

L'homme du métier ajustera facilement eu égard à la réaction mise en jeu, la quantité d'agent d'ammonolyse nécessaire pour obtenir l'amine tertiaire souhaitée.

Suivant la nature de l'agent d'ammonolyse utilisée, le bilan réactionnel varie.

Lorsque l'agent d'ammonolyse est l'ammoniac, l'équation du bilan réactionnel s'écrit :

$$(1) \qquad NH_3 + 3\ HO\text{-}A\text{-}O\text{-}(B\text{-}O)_n\text{-}R \rightarrow 3\ H_2O + N[A\text{-}O\text{-}(B\text{-}O)_n\text{-}R]_3$$

Lorsque l'agent d'ammonolyse est une amine primaire de formule (I'), l'équation du bilan réactionnel s'écrit :

$$(2) \qquad NH_2[A\text{-}O\text{-}(B\text{-}O)_n\text{-}R] + 2\ HO\text{-}A\text{-}O\text{-}(B\text{-}O)_n\text{-}R$$

$$\rightarrow N[A\text{-}O\text{-}(B\text{-}O)_n\text{-}R]_3 + 2\ H_2O$$

Lorsque l'agent d'ammonolyse est une amine secondaire de formule (I'), l'équation du bilan réactionnel s'écrit :

$$(3) \qquad NH[A\text{-}O\text{-}(B\text{-}O)_n\text{-}R]_2 + 2\ HO\text{-}A\text{-}O\text{-}(B\text{-}O)_n\text{-}R$$

$$\rightarrow N[A\text{-}O\text{-}(B\text{-}O)_nR]_3 + H_2O$$

De façon générale, la solution de réactifs contiendra de 0,5 à 2 moles d'agents d'ammonolyse par litre du monoéther d'alkylèneglycol, de préférence de 1 à 2 moles/l.

Lorsque la solution des réactifs est préparée par injection d'ammoniac gazeux dans un courant liquide entrant d'une solution de monoéther d'alkylèneglycol, en entrée du lit catalytique, les débits respectifs de l'ammoniac gazeux et de la solution de monoéther d'alkylèneglycol sont réglés de façon à préparer une solution de réactifs présentant d'environ 0,5 mole à environ 2 moles d'ammoniac par litre du monoéther d'alkylèneglycol, de préférence de 1 mole à 2 mol/l.

Le lit catalytique est constitué d'un amoncellement de particules d'un catalyseur d'hydrogénation-déshydrogénation. La forme des particules de catalyseurs n'est pas critique selon l'invention dès lors que l'amoncellement desdites particules permet néanmoins le passage d'un liquide.

Les particules de catalyseur peuvent notamment se présenter sous la forme de pastilles, d'extrudats ou de granulés éventuellement poreux ou encore de billes ou de plaquettes. De préférence, le diamètre moyen équivalent des particules est compris entre 3 mm et 15 mm. On entend par diamètre moyen équivalent des particules de catalyseur, le diamètre moyen dans le cas de particules sous forme de billes pleines, et le diamètre de la bille pleine de masse équivalente à celle de la particule dans le cas des autres types de particules.

De préférence, la surface spécifique du catalyseur est comprise entre 2 et 200 $m^2$/g.

Le catalyseur d'hydrogénation-déshydrogénation proprement dit est du même type que ceux utilisés dans le cadre de la demande EP18884. Ces catalyseurs sont par exemple à base de nickel (0), de cobalt (0), de chrome (0)

ou d'un mélange de ces métaux. Ils peuvent contenir une certaine proportion de ces mêmes métaux à un état d'oxydation différent et ceci de façon à réduire leurs caractéristiques pyrophoriques. En variante, le catalyseur est à base d'un oxyde de nickel, d'un oxyde de cobalt, d'un oxyde de chrome ou d'un mélange de tels oxydes. Le catalyseur peut également comprendre un ou plusieurs des oxydes métalliques définis ci-dessus en association avec du nickel (0), du cobalt (0) et/ou du chrome (0). Le catalyseur d'hydrogénation-déshydrogénation peut être déposé sur un support inerte tel que la silice, l'oxyde de magnésium, l'alumine, le kieselguhr ou l'oxyde de titane. Les catalyseurs contenant du cuivre ne sont pas utilisables selon l'invention.

De tels catalyseurs sont couramment disponibles dans le commerce. On peut citer le catalyseur à base de nickel Ni 563 commercialisé par la société Procatalyse et les catalyseurs à base de nickel Ni 3266 et Ni 5124 commercialisés par la société Harshaw Chemical Company. Ces catalyseurs se présentent sous la forme de pastilles (pleines

ou creuses) dont le diamètre peut à la demande être compris entre 3 mm et 20 mm. La masse volumique apparente de telles pastilles est de l'ordre de 1 à 1,5 tonne/$m^3$. La teneur en espèce catalysante est comprise suivant les variétés entre 40 et 90 % en poids. Plus précisément, le catalyseur Ni 563 est un catalyseur à base de nickel déposé sur un support de silice. Dans ce catalyseur, le rapport massique Ni/(Ni + $SiO_2$) est d'environ 80 %. Ce catalyseur est utilisé sous forme réduite. Sous cette forme, le rapport massique Ni/NiO est voisin de 50/50, ce rapport pouvant varier notablement sans modifier les performances du catalyseur.

Dans le cadre de l'invention, les catalyseurs à base de nickel sont préférés.

De manière préférée, le lit catalytique est maintenu en position verticale, la solution de réactif circulant du haut vers le bas ou du bas vers le haut. De façon avantageuse, la circulation de la solution des réactifs est effectuée du haut vers le bas. Toutefois, la disposition du lit catalytique et le sens de circulation de la solution au sein du lit catalytique ne sont pas critiques selon l'invention.

Les dimensions du lit catalytique sont à ajuster en fonction des productions désirées. Toutefois, la section du lit catalytique, perpendiculaire au flux de liquide traversant le lit catalytique, ne doit pas être trop importante de façon à assurer un contact optimum entre le catalyseur et le flux de liquide. Le diamètre optimum de cette section dépend du diamètre moyen équivalent du catalyseur et du débit à traiter. Pour le calcul du diamètre optimum, l'homme du métier pourra par exemple se rapporter au "Chemical reactor omnibook de Levenspiel ; OSU Book Stores Inc.".

Selon invention, on entend par vitesse spatiale, le rapport du débit de la solution de réactifs exprimé en tonne/h, à la masse totale du lit catalytique, exprimé en tonne.

De manière avantageuse, on maintiendra la vitesse spatiale entre 0,1 et 0,5 $h^{-1}$ lorsque le diamètre équivalent des particules est compris entre 3 et 15 mm.

Selon un mode de réalisation préféré de l'invention, le lit catalytique est un tube garni desdites particules de catalyseur d'hydrogénation-déshydrogénation. En variante, il est possible de former le lit catalytique en disposant en série plusieurs tubes garnis tels que définis ci-dessus.

La réaction de l'agent d'ammonolyse sur le monoéther d'alkylèneglycol est de préférence conduite sous pression atmosphérique. Toutefois, il est possible de conduire la réaction sous pression d'ammoniac ou sous pression d'hydrogène. De manière générale, la pression sera maintenue entre 1 et 15 atmosphères.

Il est particulièrement avantageux d'opérer en présence d'hydrogène pendant la réaction de l'agent d'ammonolyse sur le monoéther d'alkylèneglycol et ceci de façon à prolonger la durée de vie du catalyseur.

Pour ce faire, on peut placer le lit catalytique sous pression d'hydrogène ou bien faire circuler un flux d'hydrogène au travers du lit catalytique pendant que la solution de réactifs traverse le lit catalytique.

Le procédé de l'invention peut être mis en oeuvre de façon continue ou semi-continue.

Une façon préférée d'opérer en continu consiste à alimenter en continu le lit catalytique d'une solution des réactifs préalablement portée à une température de 100 à 250°C (de préférence de 100 à 180°C) recycler une partie de la solution sortant du lit catalytique à l'entrée du lit catalytique, ou préférablement, en une position intermédiaire située entre l'entrée et la sortie du lit catalytique. 15

De préférence, 50 % à 70 % de la solution sortant du lit catalytique est recyclée en tête de colonne.

Lorsqu'on opère de façon semi-continue, la solution sortant du lit catalytique est récupérée dans un bac de récupération, équipé ou non d'un système d'agitation; jusqu'à ce que la teneur en tris(éther-amine) de formule (I) soit comprise entre 0,5 et 2 moles par litre dans le bac de récupération. Lorsque cette concentration est atteinte, l'ensemble de la solution contenue dans le bac de récupération est recyclé en tête du lit catalytique et, parallèlement, l'alimentation du lit catalytique en solution de réactifs est stoppée. Suivant le degré de conversion souhaité du monoéther d'alkylèneglycol (II) en tris(éther-amine) de formule (I), on peut répéter l'opération de recyclage de la solution récupérée dans le bac de récupération à plusieurs reprises,

Aussi l'invention concerne également un procédé comprenant les étapes consistant à :

(i) alimenter en continu le lit catalytique d'une solution des réactifs, préalablement portée à une température de 100 à 250°C, et récupérer en sortie dudit lit catalytique la solution traitée dans un bac de récupération jusqu'à

obtenir une concentration en tris(éther-amine) de formule (I) de 0,5 à 2 mol/l dans le bac de récupération : puis,
(ii) recycler la solution récupérée dans ledit bac de récupération vers le lit catalytique; et la faire traverser à nouveau le lit catalytique, et
(iii) répéter l'étape de recyclage de la solution récupérée dans le bac de récupération, à l'issue de l'étape (ii), vers le lit catalytique le nombre de fois nécessaire pour obtenir le degré de conversion désiré en amine tertiaire de formule (I).

De préférence, on alimente le lit catalytique avec une solution des réactifs à une température de 100 à 180°C, mieux encore de 150 à 175°C.

Il n'est pas souhaitable de viser un degré de conversion en amine tertiaire de formule (I) supérieur à 50-70% étant donné qu'il est facile de séparer l'amine tertiaire du mélange réactionnel brut. En effet, la vitesse de réaction diminue avec l'avancement de la réaction et parallèlement la proportion d'amine secondaire diminue.

L'homme du métier pourra également isoler les amines intermédiaires primaire et secondaire du milieu réactionnel et les utiliser comme agent d'ammonolyse pour une autre réaction. L'un des sous-produits de la réaction est l'eau. Celle-ci se sépare très facilement du produit de la réaction.

Quel que soit le mode de fonctionnement sélectionné (continu ou semi-continu), on récupère en sortie du lit catalytique un flux gazeux contenant un mélange d'ammoniac et d'eau comprenant une certaine quantité de monoéther d'alkylèneglycol n'ayant pas réagi.

Par simple condensation de ce gaz, on obtient un liquide riche en ammoniac duquel on peut séparer l'eau, l'ammoniac et le monoéther d'alkylèneglycol.

Les composés de formule (II) sont connus. Lorsque A et B sont identiques, le monoéther d'alkylèneglycol peut être préparé simplement par réaction d'un alcool de formule (III) :

$$R\text{-}OH \qquad (III)$$

dans laquelle R est tel que défini ci-dessus pour la formule (I), sur un oxyde d'alkylène de formule (IV):

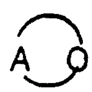 (IV)

dans laquelle A est tel que défini ci-dessus pour la formule (I).

Parmi les monoéthers d'alkylèneglycol pouvant être mis en oeuvre, on peut citer :

- l'oxa-3-butanol-1
- le dioxa-3,6-heptanol-1
- le trioxa-3,6,9-décanol-1
- l'oxa-3-pentanol-1
- le dioxa-3,6-octanol-1
- le trioxa-3,6,9-undécanol-1
- l'oxa-3-hexanol-1
- le dioxa-3,6-nonanol-1
- le trioxa-3,6,9-dodécanol-1
- l'oxa-3-heptanol-1
- le dioxa-3,6-décanol-1
- le trioxa-3,6,9-tridécanol-1
- le phénoxy-5-oxa-3-pentanol-1
- le phénoxy-8-dioxa-3,6-octanol-1
- le cyclohexoxy-5-oxa-3-pentanol-1
- le cyclohexoxy-8-dioxa-3,6-octanol-1
- le nonylphénoxy-5-oxa-3-pentanol-1
- le nonylphénoxy-8-dioxa-3,6-octanol-1
- le dodécylphénoxy-5-oxa-3-pentanol-1
- le dodécylphénoxy-8-dioxa-3,6-octanol-1
- le dioxa-3,6-méthyl-4-heptanol-1
- le dioxa-3,6-diméthyl-2,4-heptanol-1

Les aminés tertiaires de formule (I) préparées selon le procédé de l'invention permettent de solubiliser ou d'augmenter la solubilité de sels organiques ou minéraux dans les solvants organiques. Elles sont donc utiles comme agents sequestrants mais peuvent également être utilisées comme catalyseurs, du fait de leurs excellentes propriétés complexantes, ou bien comme émulsifiants.
Par ailleurs, contrairement à la plupart des éthers couronnes cycliques, les amines de formule (I) sont peu toxiques. Ces propriétés permettent d'envisager l'utilisation de ces amines tertiaires dans des domaines très variés allant de la récupération de gaz naturel acide à la formulation de tensioactifs et à la catalyse chimique.
Le procédé de l'invention est plus particulièrement approprié à la préparation des amines tertiaires suivantes :

- la tris(oxa-3-butyl)amine
- la tris(dioxa-3,6-heptyl)amine
- la tris(trioxa-3,6,9-décyl)amine
- la tris(oxa-3-pentyl)amine
- la tris(dioxa-3,6-octyl)amine
- la tris(trioxa-3,6,9-undécyl)amine
- la tris(oxa-3-hexyl)amine
- la tris(dioxa-3,6-nonyl)amine
- la tris(trioxa-3,6,9-dodécyl)amine
- la tris(oxa-3-heptyl)amine
- la tris(dioxa-3,6-décyl)amine
- la tris(trioxa-3,6,9-tridécyl)amine
- la tris(dioxa-3,6-méthyl-4-heptyl)amine
- la tris(dioxa-3,6-diméthyl-2,4-heptyl)amine
- la tris(phénoxy-5-oxa-3-pentyl)amine
- la tris(phénoxy-8-dioxa-3,6-octyl)amine
- la tris(cyclohexoxy-5-oxa-3-pentyl)amine
- la tris(cyclohexoxy-8-dioxa-3,6-octyl)amine
- la tris(nonylphénoxy-5-oxa-3-pentyl)amine
- la tris(nonylphénoxy-8-dioxa-3,6-octyl)amine
- la tris(dodécylphénoxy-5-oxa-3-pentyl)amine
- la tris(dodécylphénoxy-8-dioxa-3,6-octyl)amine

Les exemples suivants illustrent l'invention

## Exemple 1

Préparation de tris(dioxa-3,6-heptyl)amine en mode continu :
Le lit catalytique utilisé est constitué de quatre tubes, montés en série, d'un diamètre de 30 mm et d'une longueur de 3 m, remplis chacun de
2,6 kg de catalyseur Ni563 (vendu par la société Procatalyse) se présentant sous la forme de pastilles d'un diamètre moyen équivalent de 8 mm.
Les tubes de catalyseur sont maintenus en fonctionnement pseudo-adiabatique par une enveloppe de calorifuge et une série de résistances électriques noyées dans ledit calorifuge de telle sorte que la température de la paroi interne de chaque tube soit identique à la température de la paroi externe.
La solution de réactifs est constituée de monoéther méthylique de diéthylèneglycol et contient 6% en poids d'ammoniac. On alimente la tête du lit catalytique d'une solution de réactifs préchauffée à 170°C à un débit de 1,4 kg/h. Cette opération est réalisée sous pression atmosphérique. La solution récupérée en sortie du lit catalytique est un liquide jaune clair contenant 59 % en poids de tris(dioxa-3,6-heptyl) amine, 3,5 % en poids de bis (dioxa-3,6-heptyl) amine et 2,5 % en poids de mono(dioxa-3,6-heptyl)amine, le complément à 100 % en poids étant constitué de monoéther méthylique de diéthylèneglycol n'ayant pas réagi.
Une distillation sous pression réduite (666,5 Pa, soit 5 mm de Hg) poussée jusqu'à 240°C révèle la formation de moins de 1% en poids de goudrons.

## Exemple 2

Préparation de tris(dioxa-3,6-heptyl)amine en mode continu:
Cet exemple est réalisé en utilisant un protocole opératoire identique à celui de l'exemple 1, sinpn que le débit de la solution de monoéther méthylique de diéthylèneglycol à travers le lit catalytique a été fixé à 2,8 kg/h.

Dans ces conditions, on récupère en sortie du lit catalytique:

- mono(dioxa-3,6-heptyl)amine : 0,3 %
- bis(dioxa-3,6-heptyl)amine : 15-17%
- tris(dioxa-3,6-heptyl)amine : 33-37 %
- [bis(dioxa-3,6-heptyl)](methoxyéthyl)amine : 1,1 %

les pourcentages indiqués étant des pourcentages en poids par rapport au poids total de la solution.
Le complément à 100 % en poids est constitué de monoéther méthylique de diéthylèneglycol n'ayant pas réagi.

## Exemple 3

Préparation de tris(dioxa-3,6-heptyl)amine en mode continu et en présence d'hydrogène :
Dans cet exemple, on utilise le même protocole opératoire qu'à l'exemple 1 sinon que le lit catalytique est maintenu en permanence sous une pression d'hydrogène de 15 atmosphères (1,52 $10^6$ Pa).
L'analyse de la solution récupérée en sortie du lit catalytique correspond exactement à celle de l'exemple 1.
Ce résultat confirme que la présence d'hydrogène ne modifie pas le produit de la réaction mais contribue uniquement à prolonger la durée de vie du catalyseur.

## Exemple 4

Préparation de tris(dioxa-3,6-heptyl)amine en mode continu :
Dans cet exemple, l'agent d'ammonolyse est la bis(dioxa-3,6-heptyl)amine. On alimente le lit catalytique décrit à l'exemple 1 d'une solution de réactifs constituée de 80 % en poids de monoéther méthylique de diéthylèneglycol et de 20 % en poids de bis(dioxa-3,6-héptyl)amine, préalablement portée à 170°C à un débit de 1,4 kg/h.
Pour le reste, les conditions opératoires correspondent à celles mises en oeuvre dans le cadre de l'exemple 1.
On récupère en sortie du lit catalytique un liquide pratiquement incolore et comprenant, en pourcentage en poids :

- bis(dioxa-3,6-heptyf)amine : 2 %
- tris(dioxa-3,6-heptyl)amine : 26 %
- [bis(dioxa-3,6-heptyl)](méthoxyéthyl)amine : 0,5 %
- monoéther méthylique de diéthylèneglycol: q.s.p. 100%

## Exemple 5

Préparation de tris(dioxa-3,6-heptyl)amine en mode continu :
Le lit catalytique utilisé est constitué de deux tubes calorifuges montés en série, d'un diamètre de 30 cm et d'une longueur de 12 m et remplis chacun de 980 kg de catalyseur Ni 563 réduit (commercialisé par la société Procatalyse) se présentant sous la forme de pastilles d'un diamètre moyen équivalent de 8 mm.
La solution de réactifs est préparée en injectant en tête du lit catalytique un flux de 18 kg/h d'ammoniac gazeux dans un courant liquide de 284 kg/h de monoéther méthylique de diéthylèneglycol préalablement porté à 170°C.
Dans le cadre de cet exemple, on opère sous pression atmosphérique.
En pied du premier tube calorifuge, on élimine, sous forme gazeuse, un mélange de vapeur d'eau et d'ammoniac.
Le liquide sortant du premier tube calorifuge est dirigé à l'entrée du deuxième tube calorifuge formant partie du lit catalytique.
En régime stabilisé, on recueille en pied du deuxième tube un courant sortant de 270 kg/h constitué de (en pourcentage en poids):

- tris(dioxa-3,6-heptyl)amine : 60 %
- bis(dioxa-3,6-heptyl)amine : 4 %
- [bis(dioxa-3,6-heptyl)][méthoxyléthyl]amine : 3 %
- monoéther méthylique de diéthylèneglycol : q.s.p 100 %.

Ce liquide peut être distillé sous pression réduite (1333 Pa, 10 mm de Hg) sans décomposition de la tris (dioxa-3,6-heptyl)amine qui est récupérée avec une pureté de 98 %.

**Exemple 6**

Préparation de tris(dioxa-3,6-heptyl)amine en mode continu :

Le lit catalytique de l'exemple 5 est alimenté par un courant de 284 kg/h de monoéther méthylique de diéthylèneglycol préalablement porté à 170°C, dans lequel sont injectés, en entrée du lit catalytique, 18 kg/h d'ammoniac gazeux.

150 kg/h du liquide sortant du lit catalytique sont recyclés en permanence à l'entrée du lit catalytique.

En régime stabilisé, on obtient un courant sortant de 270 kg/h d'un mélange ayant pratiquement la même composition qu'à l'exemple 5.

**Exemple 7**

Préparation de tris(dioxa-3,6-heptyl)amine en mode continu

Dans cet exemple, on utilise le même protocole opératoire qu'à l'exemple 5 sinon que le deuxième tube calorifuge formant le lit catalytique est maintenu en permanence sous une pression d'hydrogène de 5 atmosphères ($5,065.10^5$ Pa).

La consommation globale d'hydrogène est de 0,4 $m^3$ TPN/h.

L'analyse de la solution en sortie du deuxième tube calorifuge correspond exactement à celle obtenue à l'exemple 5.

**Revendications**

1. Procédé de préparation de tris (éther-amines) de formule (I)

$$\text{(I)} \qquad N[A\text{-}O\text{-}(B\text{-}O)_n\text{-}R]_3$$

dans laquelle:

R représente un radical alkyle présentant de 1 à 24 atomes de carbone, éventuellement substitué par un ou plusieurs radicaux alcoxy en $C_1$-$C_{12}$ ; un radical carbocyclique, monocyclique ou polycyclique, saturé, présentant de 3 à 10 atomes de carbone, éventuellement substitué par un ou plusieurs radicaux alcoxy en $C_1$-$C_{12}$ ; un radical alkyle présentant de 1 à 12 atomes de carbone et porteur d'un groupe carbocyclique, monocyclique ou polycyclique, saturé, présentant de 3 à 10 atomes de carbone, la partie alkyle étant éventuellement substituée par un ou plusieurs radicaux alcoxy en $C_1$-$C_{12}$ et le groupe carbocyclique saturé étant éventuellement substitué par un ou plusieurs groupes alkyle en $C_1$-$C_{12}$ ou alcoxy en $C_1$-$C_{12}$ ; un radical carbocyclique aromatique, monocyclique ou polycyclique, présentant de 6 à 22 atomes de carbone et éventuellement substitué par un ou plusieurs groupes $C_1$-$C_{12}$ alcoxy ou $C_1$-$C_{12}$ alkyle ; et un radical alkyle présentant de 1 à 12 atomes de carbone et porteur d'un groupe carbocyclique aromatique, monocyclique ou polycyclique, ayant de 6 à 18 atomes de carbone, la partie alkyle étant éventuellement substituée par un ou plusieurs groupes alcoxy en $C_1$-$C_{12}$ et le groupe aromatique étant éventuellement substitué par un ou plusieurs alkyle en $C_1$-$C_{12}$ ou alcoxy en $C_1$-$C_{12}$ ;
A et B, identiques ou différents, représentent indépendamment une chaîne alkylène linéaire présentant de 1 à 24 atomes de carbone éventuellement substituée par un ou plusieurs groupes choisis parmi alkyle en $C_1$-$C_{12}$ et alcoxy en $C_1$-$C_{12}$ ; et
n représente de 0 à 12 ;

ledit procédé comprenant la réaction, en phase liquide, d'un monoéther d'alkylèneglycol de formule (II) :

$$\text{(II)} \qquad HO\text{-}A\text{-}O\text{-}(B\text{-}O)_n\text{-}R$$

où R, A, B et n sont tels que définis ci-dessus pour la formule (I), avec un agent d'ammonolyse choisi parmi l'ammoniac et une éther-amine de formule (I') :

$$\text{(I')} \qquad H_{3\text{-}p}N\ [A\text{-}O\text{-}(B\text{-}O)_n\text{-}R]_p$$

où A, B, n et R sont tels que définis ci-dessus pour la formule (I) et p représente 1 ou 2, à une température comprise entre 100 et 250°C, par mise en contact des réactifs avec un catalyseur d'hydrogénation-déshydrogénation, **caractérisé en ce que** la mise en contact est réalisée en faisant passer une solution desdits réactifs préalablement portée à une température de 100 à 250°C à travers un lit catalytique constitué de particules dudit catalyseur d'hydrogénation-déshydrogénation.

**2.** Procédé selon la revendication 1, **caractérisé en ce que** :

R représente $(C_1\text{-}C_{24})$alkyle, cyclohexyle, phényle ou $(C_1\text{-}C_{12})$ alkylphényle;
A et B identiques ou différents, représentent indépendamment une chaîne $(C_2\text{-}C_3)$ alkylène linéaire dans laquelle les atomes de carbone sont éventuellement substitués par méthyle ou éthyle; et
n représente un nombre entier compris entre 0 et 4.

**3.** Procédé selon l'une quelconque des revendications précédentes, **caractérisé en ce que** l'on porte la solution desdits réactifs à une température de 100 à 180°C avant de la faire passer à travers ledit lit catalytique.

**4.** Procédé selon l'une quelconque des revendications précédentes, **caractérisé en ce que** les particules de catalyseurs se présentent sous la forme de pastilles, d'extrudats ou de granulés éventuellement poreux ou encore de billes ou de plaquettes.

**5.** Procédé selon la revendication 4, **caractérisé en ce que** le diamètre moyen équivalent des particules de catalyseur est compris entre 3 et 15 mm.

**6.** Procédé selon l'une quelconque des revendications précédentes, **caractérisé en ce que** le catalyseur d'hydrogénation-déshydrogénation est à base d'un composé choisi parmi le nickel(0), le cobalt (0), le chrome (0), un oxyde de nickel, un oxyde de cobalt, un oxyde de chrome et un de leurs mélanges.

**7.** Procédé selon l'une quelconque des revendications précédentes, **caractérisé en ce que** les particules de catalyseur sont constituées d'un catalyseur d'hydrogénation-déshydrogénation supporté sur un support inerte tel que la silice, l'oxyde de magnésium, l'alumine, le kieselguhr ou l'oxyde de titane.

**8.** Procédé selon l'une quelconque des revendications précédentes, **caractérisé en ce que** l'on fait passer la solution des réactifs à travers le lit catalytique à une vitesse spatiale, définie comme étant le rapport du débit de ladite solution, exprimé en tonne/h, à la masse totale du lit catalytique, exprimé en tonne, comprise entre 0,1 et 0,5 $h^{-1}$.

**9.** Procédé selon l'une quelconque des revendications précédentes, **caractérisé en ce que** la solution desdits réactifs comprend de 0,5 à 2 moles de l'agent d'ammonolyse par litre du monoéther d'alkylèneglycol (II).

**10.** Procédé selon l'une quelconque des revendications précédentes, **caractérisé en ce que** la solution des réactifs est préparée en injectant, à l'entrée du lit catalytique l'ammoniac gazeux dans un courant liquide entrant d'une solution dudit monoéther d'alkylèneglycol (II), préalablement portée à une température de 100 à 250°C.

**11.** Procédé selon l'une quelconque des revendications précédentes, **caractérisé en ce qu'**il est réalisé en continu.

**12.** Procédé selon l'une quelconque des revendications précédentes, **caractérisé en ce qu'**une partie de la solution sortant du lit catalytique est recyclée à l'entrée dudit lit catalytique ou en une position intermédiaire située entre l'entrée et la sortie du lit catalytique.

**13.** Procédé selon l'une quelconque des revendications 1 à 10, **caractérisé en ce que** le procédé est réalisé de façon semi-continue par mise en oeuvre des étapes consistant à :

(i) alimenter en continu le lit catalytique d'une solution des réactifs, préalablement portée à une température de 100 à 250°C, et récupérer en sortie dudit lit catalytique la solution traitée dans un bac de récupération jusqu'à obtenir une concentration en tris(éther-amine) de formule (I) de 0,5 à 2 mol/l dans le bac de récupération, puis
(ii) recycler la solution récupérée dans ledit bac de récupération vers le lit catalytique; et la faire traverser à nouveau le lit catalytique et
(iii) répéter l'étape de recyclage de la solution récupérée dans le bac de récupération, à l'issue de l'étape (ii),

vers le lit catalytique le nombre de fois nécessaire pour obtenir le degré de conversion désiré en amine tertiaire de formule (I).

**14.** Procédé selon l'une quelconque des revendications, **caractérisé en ce que** le lit catalytique est maintenu en position verticale, la circulation de la solution des réactifs étant effectuée du haut vers le bas.

**15.** Procédé selon l'une quelconque des revendications précédentes, **caractérisé en ce que** l'on fait réagir l'ammoniac sur l'éther monométhylique du diéthylèneglycol pour la préparation de tris(dioxa-3,6-heptyl)amine.

**Patentansprüche**

**1.** Verfahren zur Herstellung von Tris(etheraminen) der Formel (I)

$$\text{(I)} \qquad N[A\text{-}O\text{-}(B\text{-}O)_n\text{-}R]_3$$

worin:

Reinen 1 bis 24 Kohlenstoffatome aufweisenden Alkylrest, der gegebenenfalls durch einen oder mehr $C_1$-$C_{12}$-Alkoxyreste substituiert ist; einen carbocyclischen, monocyclischen oder polycyclischen, gesättigten, 3 bis 10 Kohlenstoffatome aufweisenden Rest, der gegebenenfalls durch einen oder mehr $C_1$-$C_{12}$-Alkoxyreste substituiert ist; einen 1 bis 12 Kohlenstoffatome aufweisenden Alkylrest, der eine carbocyclische, monocyclische oder polycyclische, gesättigte, 3 bis 10 Kohlenstoffatome aufweisende Gruppe trägt, wobei der Alkylteil gegebenenfalls durch einen oder mehr $C_1$-$C_{12}$-Alkoxyreste substituiert ist und die gesättigte carbocyclische Gruppe gegebenenfalls durch eine oder mehr $C_1$-$C_{12}$-Alkyl- oder $C_1$-$C_{12}$-Alkoxygruppen substituiert ist; einen carbocyclischen aromatischen, monocyclischen oder polycyclischen, 6 bis 22 Kohlenstoffatome aufweisenden Rest, der gegebenenfalls durch eine oder mehr $C_1$-$C_{12}$-Alkoxy- oder $C_1$-$C_{12}$-Alkylgruppen substituiert ist; und einen Alkylrest darstellt, der 1 bis 12 Kohlenstoffatome aufweist und eine carbocyclische aromatische, monocyclische oder polycyclische Gruppe mit 6 bis 18 Kohlenstoffatomen trägt, wobei der Alkylteil gegebenenfalls durch eine mehr $C_1$-$C_{12}$-Alkoxygruppen substituiert ist und die aromatische Gruppe gegebenenfalls durch ein oder mehr $C_1$-$C_{12}$-Alkyl oder $C_1$-$C_{12}$-Alkoxy substituiert ist;
A und B, die gleich oder verschieden sind, unabhängig eine gerade Alkylenkette darstellen, die 1 bis 24 Kohlenstoffatome aufweist, die gegebenenfalls durch eine oder mehr aus $C_1$-$C_{12}$-Alkyl und $C_1$-$C_{12}$-Alkoxy ausgewählte Gruppen substituiert ist, und
n unabhängig 0 bis 12 darstellt,

wobei das Verfahren die Reaktion eines Alkylenglykolmonoethers der Formel (II):

$$\text{(II)}\ HO\text{-}A\text{-}O\text{-}(B\text{-}O)_n\text{-}R$$

worin R, A, B und n wie vorstehend für die Formel (I) definiert sind, in flüssiger Phase mit einem Mittel zur Ammonolyse, das aus Ammoniak und einem Etheramin der Formel (I') ausgewählt ist:

$$\text{(I')}\ H_{3\text{-}p}N[A\text{-}O\text{-}(B\text{-}O)_n\text{-}R]_p$$

worin A, B, n und R wie vorstehend für die Formel (I) definiert sind und p 1 oder 2 darstellt, bei einer Temperatur zwischen 100 und 250°C durch In-Kontakt-bringen der Reaktanten mit einem Hydrierungs-/Dehydrierungskatalysator umfaßt, **dadurch gekennzeichnet, daß** das In-Kontakt-bringen dadurch ausgeführt wird, daß man eine zuvor auf eine Temperatur von 100 bis 250°C gebrachte Lösung der Reaktanten durch ein Katalysatorbett laufen läßt, das aus Teilchen des Hydrierungs-/Dehydrierungskatalysators besteht.

**2.** Verfahren gemäß Anspruch 1, **dadurch gekennzeichnet, daß**

R ($C_1$-$C_{24}$)Alkyl, Cyclohexyl, Phenyl oder ($C_1$-$C_{12}$)Alkylphenyl darstellt,
A und B, die gleich oder verschieden sind, unabhängig eine gerade ($C_2$-$C_3$)Alkylenkette darstellen, worin die

Kohlenstoffatome gegebenenfalls durch Methyl oder Ethyl substituiert sind, und
n eine ganze Zahl zwischen 0 und 4 darstellt.

**3.** Verfahren gemäß einem der vorangehenden Ansprüche, **dadurch gekennzeichnet, daß** man die Lösung der Reaktanten auf eine Temperatur von 100 bis 180°C bringt, bevor man sie durch das Katalysatorbett laufen läßt.

**4.** Verfahren gemäß einem der vorangehenden Ansprüche, **dadurch gekennzeichnet, daß** sich die Katalysatorteilchen in Form von Pellets, Extrudaten oder gegebenenfalls porösem Granulat oder auch Kugeln oder Plättchen befinden.

**5.** Verfahren gemäß Anspruch 4, **dadurch gekennzeichnet, daß** der mittlere äquivalente Durchmesser der Katalysatorteilchen zwischen 3 und 15 mm ist.

**6.** Verfahren gemäß einem der vorangehenden Ansprüche, **dadurch gekennzeichnet, daß** der Hydrierungs-/Dehydrierungskatalysator auf einer Verbindung beruht, die aus Nickel(0), Kobalt(0), Chrom(0), einem Nickeloxid, einem Kobaltoxid, einem chromoxid oder einem ihrer Gemische ausgewählt ist.

**7.** Verfahren gemäß einem der vorangehenden Ansprüche, **dadurch gekennzeichnet, daß** die Katalysatorteilchen aus einem Hydrierungs-/Dehydrierungskatalysator bestehen, der auf einem inerten Träger wie etwa Siliziumoxid, Magnesiumoxid, Aluminiumoxid, Kieselgur oder Titanoxid geträgert ist.

**8.** Verfahren gemäß einem der vorangehenden Ansprüche, **dadurch gekennzeichnet, daß** man die Lösung der Reaktanten mit einer Raumgeschwindigkeit, die durch das Verhältnis des in Tonnen/h ausgedrückten Durchsatzes der Lösung zu der in Tonnen ausgedrückten Gesamtmasse des Katalysatorbetts definiert ist, zwischen 0,1 und 0,5 $h^{-1}$ durch das Katalysatorbett fließen läßt.

**9.** Verfahren gemäß einem der vorangehenden Ansprüche, **dadurch gekennzeichnet, daß** die Lösung der Reaktanten 0,5 bis 2 Mol Ammonolysereagenz je Liter Ethylenglykolmonoether (I) umfaßt.

**10.** Verfahren gemäß einem der vorangehenden Ansprüche, **dadurch gekennzeichnet, daß** die Lösung der Reaktanten dadurch hergestellt, daß man am Eingang des Katalysatorbetts gasförmigen Ammoniak in einen eintretenden Flüssigkeitsstrom aus einer zuvor auf eine Temperatur von 100 bis 250°C gebrachten Lösung des Alkylenglykolmonethers (II) einbläst.

**11.** Verfahren gemäß einem der vorangehenden Ansprüche, **dadurch gekennzeichnet, daß** es kontinuierlich durchgeführt wird.

**12.** Verfahren gemäß einem der vorangehenden Ansprüche, **dadurch gekennzeichnet, daß** ein Teil der aus dem Katalysatorbett austretenden Flüssigkeit zum Eingang des Katalysatorbetts oder einer zwischen dem Eingang und dem Ausgang des Katalysatorbetts gelegenen Stelle zurückgeführt wird..

**13.** Verfahren gemäß einem der Ansprüche 1 bis 10, **dadurch gekennzeichnet, daß** das Verfahren in halbkontinuierlicher Weise durch Ausführen der Schritte durchgeführt wird umfassend:

(i) das ununterbrochene Versorgen des Katalysatorbetts mit einer zuvor auf eine Temperatur von 100 bis 250°C gebrachten Lösung der Reaktanten und das Rückgewinnen der behandelten Lösung am Ausgang des Katalysatorbetts in einem Rückgewinnungsgefäß bis zum Erhalt einer Konzentration an Tris(etheramin) der Formel (I) von 0,5 bis 2 Mol/1 in dem Rückgewinnungsgefäß, anschließend
(ii) das Rückführen der zurückgewonnenen Lösung in dem Rückgewinnungsgefäß zu dem Katalysatorbett und ihr erneutes Durchfließenlassen des Katalysatorbetts und
(iii) das Wiederholen des Rückführschrittes der zurückgewonnenen Lösung in dem Rückgewinnungsgefäß am Ende von Schritt (ii) zu dem Katalysatorbett so oft wie es zum Erhalt des gewünschten Umwandlungsgrades in das tertiäre Amin der Formel (I) notwendig ist.

**14.** Verfahren gemäß einem der vorangehenden Ansprüche, **dadurch gekennzeichnet, daß** das Katalysatorbett in senkrechter Lage gehalten wird und der Kreislauf der Reaktantenlösung von oben nach unten durchgeführt wird.

**15.** Verfahren gemäß einem der vorangehenden Ansprüche, **dadurch gekennzeichnet, daß** man zur Herstellung von

Tris(dioxa-3,6-heptyl)amin Ammoniak mit Diethylenglykolmonomethylether reagieren läßt.

## Claims

1. Process for preparing tris(aminoethers) of formula (I)

$$(I) \qquad N\,[A\text{-}O\text{-}\,(B\text{-}O)_n\text{-}R]_3$$

wherein:

R denotes an alkyl radical having 1 to 24 carbon atoms, optionally substituted by one or more $C_{1-12}$-alkoxy radicals; a saturated carbocyclic, monocyclic or polycyclic radical having 3 to 10 carbon atoms, optionally substituted by one or more $C_{1-12}$-alkoxy radicals; an alkyl radical having 1 to 12 carbon atoms and carrying a saturated carbocyclic, monocyclic or polycyclic radical having 3 to 10 carbon atoms, the alkyl moiety optionally being substituted by one or more $C_{1-12}$-alkoxy radicals and the saturated carbocyclic group optionally being substituted by one or more $C_{1-12}$-alkyl or $C_{1-12}$-alkoxy groups; an aromatic carbocyclic, monocyclic or polycyclic radical having 6 to 22 carbon atoms and optionally substituted by one or more $C_{1-12}$-alkoxy or $C_{1-12}$-alkyl groups; and an alkyl radical having 1 to 12 carbon atoms and carrying an aromatic carbocyclic, monocyclic or polycyclic radical having 6 to 18 carbon atoms, the alkyl moiety optionally being substituted by one or more $C_{1-12}$-alkoxy groups and the aromatic group optionally being substituted by one or more $C_{1-12}$-alkyl or $C_{1-12}$-alkoxy groups;
A and B, which may be identical or different, independently represent a straight alkylene chain having 1 to 24 carbon atoms optionally substituted by one or more groups selected from among $C_{1-12}$-alkyl and $C_{1-12}$-alkoxy; and
n represents from 0 to 12;

said process comprising reacting, in a liquid phase, an alkyleneglycol monoether of formula (II):

$$(II)\ HO\text{-}A\text{-}O\text{-}(B\text{-}O)_n\text{-}R$$

wherein R, A, Band n are as defined for formula (I) hereinbefore, with an ammonolysis agent selected from ammonia and an aminoether of formula (I'):

$$(I')\ H_{3-p}N[A\text{-}O\text{-}(B\text{-}O)_n\text{-}R]_p$$

wherein A, B, n and R are as defined for formula (I) hereinbefore and p denotes 1 or 2, at a temperature of between 100 and 250°C, by contacting the reagents with a hydrogenation/dehydrogenation catalyst, **characterised in that** the contacting is carried out by passing a solution of the reagents which has previously been brought to a temperature of 100 to 250°C through a bed of catalyst consisting of particles of said hydrogenation/dehydrogenation catalyst.

2. Process according to claim 1, **characterised in that**:

R denotes $(C_{1-24})$alkyl, cyclohexyl, phenyl or $(C_{1-12})$ alkylphenyl;
A and B which may be identical or different independently denote a straight $(C_{2-3})$alkylene chain wherein the carbon atoms are optionally substituted by methyl or ethyl; and
n denotes an integer between 0 and 4.

3. Process according to any one of the preceding claims, **characterised in that** the solution of the reagents is brought to a temperature of 100 to 180°C before being passed through said bed of catalyst.

4. Process according to any one of the preceding claims, **characterised in that** the catalyst particles are present in the form of pellets, extruded material or granules, which are optionally porous, or in the form of beads or flakes.

5. Process according to claim 4, **characterised in that** the mean equivalent diameter of the catalyst particles is between 3 and 15 mm.

6. Process according to any one of the preceding claims, **characterised in that** the hydrogenation/dehydrogenation catalyst is based on a compound selected from among nickel(0), cobalt (0), chromium(0), a nickel oxide, a cobalt oxide, a chrome oxide and one of the mixtures thereof.

7. Process according to any one of the preceding claims, **characterised in that** the particles of catalyst consist of a hydrogenation/dehydrogenation catalyst supported on an inert support such as silica, magnesium oxide, alumina, kieselguhr or titanium oxide.

8. Process according to any one of the preceding claims, **characterised in that** the solution of the reagents is passed through the bed of catalyst at a spatial velocity, defined as being the ratio of the flow rate of said solution, expressed in tonnes per hour, to the total mass of the bed of catalyst, expressed in tonnes, of between 0.1 and 0.5 $h^{-1}$.

9. Process according to any one of the preceding claims, **characterised in that** the solution of the reagents comprises from 0.5 to 2 moles of the ammonolysis agent per litre of the alkyleneglycol monoether (II).

10. Process according to any one of the preceding claims, **characterised in that** the solution of the reagents is prepared by injecting gaseous ammonia into an inward liquid flow of a solution of said alkyleneglycol monoether (II), previously heated to a temperature of 100 to 250°C, at the entrance to the bed of catalyst.

11. Process according to any one of the preceding claims, **characterised in that** it is carried out continuously.

12. Process according to any one of the preceding claims, **characterised in that** some of the solution leaving the bed of catalyst is recycled into the entrance to said bed of catalyst or at an intermediate position located between the entrance to and the exit from the bed of catalyst.

13. Process according to any one of claims 1 to 10, **characterised in that** the process is carried out semicontinously by performing steps consisting of:

    (i) continuously supplying the bed of catalyst with a solution of the reagents, previously heated to a temperature of 100 to 250°C, and recovering the treated solution in a recovery tank at the exit from said bed of catalyst until a concentration of tris(aminoether) of formula (I) of from 0.5 to 2 mol/l is obtained in the recovery tank, then
    (ii) recycling the recovered solution in said recovery tank into the bed of catalyst; and passing it once more through said bed of catalyst; and
    (iii) repeating the step of recycling the recovered solution in said recovery tank, at the end of step (ii), into the bed of catalyst the number of times needed to obtain the desired degree of conversion into a tertiary amine of formula (I).

14. Process according to any one of the claims,
**characterised in that** the catalytic bed is held in the vertical position, the solution of the reagents being circulated from the top towards the bottom.

15. Process according to any one of the preceding claims, **characterised in that** ammonia is reacted with diethyleneglycol monomethyl ether to prepare tris(dioxa-3,6-heptyl) amine.